# EUROPÄISCHE PATENTSCHRIFT

(11) **EP 1 087 991 B1**
(45) Veröffentlichungstag und Bekanntmachung des Hinweises auf die Patenterteilung: **02.09.2009**
(21) Anmeldenummer: 99932721.6
(22) Anmeldetag: 24.06.1999
(51) Int. Cl.: C07K 5/078, C07K 5/083, C07K 5/097, A61K 38/05, A61K 38/06

(54) **PRODRUGS VON DIPEPTIDYLPEPTIDASE IV-INHIBITOREN**
PRODRUGS OF DIPEPTIDYL PEPTIDASE IV INHIBITORS
PROMEDICAMENTS D'INHIBITEURS DE LA DIPEPTIDYLPEPTIDASE IV

(30) Priorität: 24.06.1998 DE 19828113
(43) Veröffentlichungstag der Anmeldung: 04.04.2001
(73) Patentinhaber: Prosidion Limited, Oxford, OX4 6LT (GB)
(72) Erfinder: DEMUTH, Hans-Ulrich, D-06120 Halle (DE); HOFFMANN, Torsten, D-06114 Halle (DE); SCHLENZIG, Dagmar, D-06114 Halle (DE); MANHART, Susanne, D-06110 Halle (DE)
(74) Vertreter: Blakey, Alison Jane
(86) Internationale Anmeldenummer: PCT/EP1999/004382
(87) Internationale Veröffentlichungsnummer: WO 1999/067278

(56) Entgegenhaltungen:
- WO-A-95/29691
- WO-A-98/22494
- DE-A- 19 616 486
- DEMUTZ H.-U.: 'RECENT DEVELOPMENTS IN INHIBITING CYSTEINE AND SERINE PROTEASES' JOURNAL OF ENZYME INHIBITION Bd. 3, 1990, UK, Seiten 249 - 278, XP002041620

## Beschreibung

Erfindungsgemäß werden Prodrug-Verbindungen von Inhibitoren der Dipeptidyl Peptidase IV (DP IV) bereitgestellt, welche Prodrug-Verbindungen die allgemeine Formel A-B-C aufweisen wie sie in Anspruch 1 definiert ist, wobei
A eine Aminosäure ist,
B Prolin, Hydroxyprolin, Thiazolidincarbonsäure, Dehydroprolin, Pipecolinsäure,Azetidincarbonsäure oder Aziridincarbonsäure, gebunden an A und C über Peptidbindungen, ist, und
C ein Aminoacylpyrrolidin-, Aminoacylthiazolidin- oder N-Dipeptidyl-O-acylhydroxylamin-Inhibitor von DP IV ist.

Es wurde gefunden, daß durch Verabreichung von Inhibitoren (Effektoren) der DP IV bzw. DP IV-analoger Enzymaktivität im Blut eines Säugers, durch deren damit verbundene, temporäre Aktivitätsreduktion, in kausaler Folge die endogenen (oder zusätzlich exogen verabreichten) insulinotropen Peptide Gastric Inhibitory Polypeptide 1-42 (GIP₁₋₄₂) und Glucagon-Like Peptide Amide-1 7-36 (GLP-1₇₋₃₆) (o.a. GLP-1₇₋₃₇ oder deren Analoga) durch DP IV- und DP IV-ähnliche Enzyme vermindert abgebaut werden und damit die Konzentrationsabnahme dieser Peptidhormone bzw. ihrer Analoga verringert bzw. verzögert wird. Die durch die Wirkung von DP IV-Effektoren erzielte, erhöhte Stabilität der (endogen vorhandenen oder exogen zugeführten) Incretine oder ihrer Analoga, die damit vermehrt für die insulinotrope Stimulierung der Incretin-Rezeptoren der Langerhansschen Zellen im Pankreas zur Verfügung stehen, verändert u.a. die Wirksamkeit von körpereigenem Insulin, was eine Stimulierung des Kohlehydratstoffwechsels des behandelten Organismus nach sich zieht. Als Resultat sinkt der Blutzuckerspiegel unter die für Hyperglykämie charakteristische Glukosekonzentration im Serum des behandelten Organismus. Dadurch können mittels DP IV - Inhibitoren Stoffwechselanomalien wie Übergewicht, Glukosurie, Hyperlipidämie sowie mögliche schwere metabolische Azidosen, Diabetes mellitus, die eine Folge längerer, erhöhter Glukosekonzentrationen im Blut sind, verhindert bzw. gemildert werden [vgl. DE 196 16 486].

Mit Hilfe von DP IV - Inhibitoren kann auch das Eindringen von HIV in CD 26 (DP IV) positive Zellen experimentell verhindert werden [vergl. WAKSELMAN, M., NGUYEN, C., MAZALEYRAT, J.-P., CALLEBAUT, C., KRUST, B., HOVANESSIAN, A.G., Inhibition of HIV-1 infection f CD 26+ but not CD26- cells by a potent cyclopeptidic inhibitor of the DPP IV activity of CD 26. Abstract P 44 of the 24th European Peptide Symposium 1996].

Weiterhin wurde gefunden, daß DP IV neuroaktive Peptide wie Neuropeptid Y und CLIP in ihrer Aktivität modulieren kann [vergl. MENTLEIN, R., DAHMS, P., GRANDT, D., KRUGER,R., Proteolytic processing of neuropeptide Y and peptide YY by dipeptidyl peptidase IV. Regul. Pept. 49, 133 (1993); WETZEL, W., WAGNER, T., VOGEL, D., DEMUTH, H.-U., BALSCHUN, D., Effects of the CLIP fragment ACTH 20-24 on the duration of REM sleep episodes. Neuropeptides, 31, 41 (1997)].

Die WO 95/29691 offenbart Peptidyl-Abkömmlinge von Aryldiestern von α-Aminoalkyphosphonsäuren als Inhibitoren von DPIV, z.B. Ala-Pro^{P}(OZ)₂. Die DE 19616486 offenbart die Verbindungen Alanylpyrrolidin, Isoleucyl-thiazolidin und N-Valyl-prolyl-O-benzoylhydroxylamin. J. Enzyme Inhibition, 1990, Vol. 3, pp 249-278 offenbart die Verbindung Alanyl-prolyl-O-(4-nitrobenzoyl)hydroxylamin.

Es ist die Aufgabe der vorliegenden Erfindung, Effektoren von DP IV bereitzustellen, die im Vergleich zu bekannten Inhibitoren eine gesteigerte Wirkung aufweisen und deren Wirkung zeitlich definiert eintritt.

Diese Aufgabe wird durch die Bereitstellung von Prodrug-Verbindungen von Inhibitoren der Dipeptidyl Peptidase IV (DP IV) gelöst, welche gemäß Anspruch 1 definiert sind.

Überraschenderweise weisen derartige als Prodrugs maskierte Inhibitoren gegenüber nicht maskierten Inhibitoren eine erheblich gesteigerte Wirksamkeit auf: Werden identische Mengen von unmaskierten Inhibitoren der DP IV und von erfindungsgemäßen Prodrugverbindungen eingesetzt, so kommt es bei Wistarratten durch die Prodrug-Verbindungen zu einer Glukosetoleranzverbesserung von bis zu 75 %, siehe auch Tabelle 4.

Diese Verbesserung ist umso erstaunlicher, als gefunden wurde, daß unmaskierte Inhibitoren der DP IV zu 100 % aus dem Magen-Darmtrakt von Säugern resorbiert werden und in den vaskulären Bereich des Körpers eintreten. Es wäre also zu erwarten gewesen, daß Prodrug-Verbindungen, die normalerweise nur eine Zersetzung von oral verabreichten Verbindungen im Magen-Darmtrakt verhindern sollen, zu keiner Steigerung der Wirksamkeit der Inhibitoren führen würden. Es sei nur am Rande vermerkt, daß für einen Fachmann aufgrund dieser Tatsachen außerdem auch gar keine Veranlassung bestand, nach modifizierten Inhibitoren zu suchen, auch wenn Prodrug-Verbindungen an sich bereits bekannt waren, vgl. z.B. WO 9745117.

Gemäß der vorliegenden Erfindung werden Prodrug-Verbindungen eingesetzt, in denen B Prolin, Hydroxyprolin, Thiazolidincarbonsäure, Dehydroprolin, Pipecolinsäure, Azetidincarbonsäure oder Aziridincarbonsäure ist, wobei Prolin und Hydroxyprolin bevorzugt werden. Dabei ist B über Peptidbindungen mit A und C verknüpft.

Die erfindungsgemäßen Verbindungen weisen dabei insbesondere auch den Vorteil auf, daß die Inhibitoren der DP IV je nach individuellem Bedarf der Patienten freigesetzt werden:

Tritt eine erfindungsgemäße Prodrug-Verbindung mit einem DP IV-Molekül in Wechselwirkung, so wird sie durch das Enzym in die Gruppen A-B und den Inhibitor C gespalten. Der Inhibitor C wird das DP IV-Molekül inhibieren, so daß es keine weiteren Prodrug-Verbindungen mehr aufspalten kann. Liegen weitere DP IV-Moleküle vor, so kommt es so lange zur Spaltung von Prodrug-Verbindungen (wenn eine ausreichende Menge an Prodrug-Verbindungen verabreicht wurde), bis das letzte DP IV-Molekül inhibiert ist. Die übrigen Prodrug-Verbindungen werden nicht zersetzt und stellen somit so lange ein Inhibitordepot dar, bis die Konzentration an DP IV-Molekülen wieder ansteigt bzw. Inhibitormoleküle von der DP IV verdrängt werden bzw. Inhibitormoleküle eliminiert werden und es wiederum zu einher erneuten Spaltung der Prodrug-Verbindungen und somit zu einer Freisetzung von Inhibitoren kommt.

Die Erfindung weist also den weiteren Vorteil auf, daß jeder Organismus genau die Menge an Inhibitor freisetzen wird, die zur Inhibierung der individuell in unterschiedlicher Menge vorliegenden DP IV notwendig ist. Liegt bei einem Patienten DP IV z.B. in hohen Konzentrationen vor, so wird eine große Menge an Inhibitor freigesetzt; liegt nur eine wenig erhöhte Konzentration an DP IV vor, so wird nur eine geringe Menge an Inhibitor freigesetzt.

Weiter ist erfindungsgemäß in den Prodrug-Verbindungen C ein Aminoacylpyrrolidid, Aminoacylthiazolidid oder N-Dipeptidyl-O-acyl Hydroxylamin. Derartige Inhibitoren haben sich als besonders wirksame DP IV-Inhibitoren herausgestellt. Als Beispiele derartiger Inhibitoren können z.B. Ile-Thia, Ile-Pyr, Val-Thia und Val-Pyr genannt werden.

Die Inhibitoren (Komponente C) können erfindungsgemäß auch in Salzform vorliegen, wobei organische Salze wie Acetate, Succinate, Tartrate oder Fumarate oder anorganische Säurereste wie Phosphate oder Sulfate bevorzugt werden. Besonders bevorzugt werden Fumarate.

Insbesondere werden Verbindungen bevorzugt, in denen A-B ein Dipeptid der Formel Ile-Pro oder Gly-Pro ist.

Die vorliegende Erfindung betrifft also neue Prodrug-Verbindungen von Inhibitoren der Serinpeptidase Dipeptidyl Peptidase IV, die zur Behandlung von verschiedenen Erkrankungen insbesondere von mit Diabetes mellitus im Zusammenhang stehenden Stoffwechselerkrankungen eingesetzt werden können.

Ein weiterer Vorteil der erfindungsgemäßen Prodrug-Verbindungen besteht darin, daß der Wirkungseintritt und auch die Wirkungsdauer der DP IV-Inhibitoren durch geeignete Auswahl der Gruppen A-B zeitlich gesteuert werden kann. Insbesondere hängt die Freisetzung der Gruppen A-B aus den erfindungsgemäßen Prodrug-Verbindungen von der Natur des Aminosäurerestes von A ab: Bezüglich der Definition der Gruppe A wurde insbesondere folgende Reihenfolge der Freisetzungsgeschwindigkeit der Reste A-B aus den Prodrug-Verbindungen A-B-C durch DP IV gefunden: Ile<Val<Phe<Pro<Ala<Gly. Die Geschwindigkeitskonstanten der entsprechenden DP IV-katalysierten Freisetzungen liegen zwischen 1 s⁻¹ und 100 s⁻¹. Damit steht ein Mittel zur Verfügung, die DP IV-Inhibitoren zeitlich exakt definiert freizusetzen: Soll die Wirkung der Enzyme sofort, z.B. bei Aufnahme Glukosereicher Nahrung eintreten, so wird eine Verbindung A-B-C gewählt, die als Gruppe A z.B. die Aminosäure Gly aufweist; soll erst eine verzögerte Wirkung des Inhibitors eintreten, so kann als Gruppe A z.B. die Aminosäure Ile ausgewählt werden. DP IV-Inhibitoren können durch die erfindungsgemäßen Prodrug-Verbindungen also insbesondere nahezu verzögerungsfrei, z.B. nahezu gleichzeitig mit aufgenommenen Nahrungsmitteln durch die Dünndarmmucosa transportiert werden.

Bei der Analyse der Dosis-Wirkungs-Beziehungen des DP IV - Inhibitors Isoleucyl Thiazolidid als Modulator der Blutglukosekonzentration im Säugetierorganismus kann ein Unterschied zwischen oraler und parenteraler Applikation des Wirkstoffes an Wistarratten festgestellt werden: Bei oraler Applikation wurde eine Sättigung der Aufnahme des Wirkstoffs (gemessen an der Inhibierung des Serumenzyms) beobachtet, während bei parenteraler Applikation des Inhibitors eine vollständige Hemmung des Enzyms beobachtet wurde. Das ist in Tabelle 1 beispielhaft belegt:

**Tabelle 1: Restaktivität der DP IV gegenüber 0,4 mM des Substrates H-Gly-Pro-pNA bei 30°C, pH 7,6 und einer Ionenstärke von 0,125, nach i.v. und p.o. Gabe und in Abhängigkeit von der Dosis Isoleucyl Thiazolidid (Ile-Thia), bestimmt 30 min nach Applikation des Inhibitors.**

| **Ile-Thia Dosis bei parenteraler Gabe** | **DP IV- Aktivität in %** | **Ile-Thia Dosis bei oraler Gabe** | **DP IV- Aktivität in %** |
|---|---|---|---|
| 0 mg | 100 | 0 mg | 100 |
| 0,02 mg | 80 | 2,5 mg | 52 |
| 0,2 mg | 32 | 5,0 mg | 40 |
| 2 mg | 5 | 10 mg | 28 |
| 20 mg | 0 | 20 mg | 29 |

Da auch im Darm Enzyme, insbesondere hohe Konzentrationen an DP IV, vorhanden sind, die die abspaltbaren Gruppen von Prodrugs abspalten und damit das Medikament freisetzen können, und ferner - wie bereits erwähnt - gefunden wurde, daß DP IV Inhibitoren quantitativ aus dem Magen-Darmtrakt resorbiert werden, war zu erwarten, daß der Einsatz von Prodrug-Verbindungen von DP IV-Inhibitoren keine Verbesserung dieser Situation bringen würde.

Es war daher ausgesprochen überraschend, daß gefunden wurde, daß die erfindungsgemäßen Prodrugs von DP IV-Inhibitoren gegenüber den korrespondierenden unmaskierten DP IV - Inhibitoren eine deutlich verstärkte Glukosetoleranz im Glukosetoleranztest bewirken. Dieses Verhalten war insofern besonders überraschend als die Prodrugs - wie vorstehend erwähnt - bereits im Darm durch dort vorliegende Enzyme wie die Dipeptidylpeptidase gespalten werden können und somit genau wie die unmaskierten Inhibitoren nicht mehr für einen Transport an den Zielort zur Verfügung stehen sollten:

Sobald die Prodrug-Verbindungen durch im Darm vorliegende DP IV oder andere Enzyme gespalten werden, werden die erfindungsgemäßen Inhibitoren freigesetzt, wodurch es genau wie beim Einsatz von unmaskierten Inhibitoren zu einer Inhibierung der DP IV kommt. Folglich findet kein weiterer Abbau der Prodrug-Verbindungen durch DP IV mehr statt; alle noch unzersetzt vorliegenden oder zusätzlich zugeführten Prodrug-Verbindungen können ebenso wie überschüssige, das heißt nicht an DP IV gebundene unmaskierte Inhibitoren unzersetzt aus dem Magen-Darm-Trakt in den vaskulären Bereich eines Körpers übertreten. Dort können sie dann, wie vorstehend erwähnt, je nach individuellem Bedarf als DP IV Inhibitoren eingesetzt werden. Doch auch die im Darm an DP IV gebundenen Inhibitoren werden nach einer gewissen Zeit wieder freigesetzt und treten in den vaskulären Bereich ein.

Mit Hilfe der erfindungsgemäßen Prodrug-Verbindungen kann also auch eine erwünschte Wirkungsverstärkung in vivo erreicht werden.

Ferner kann auch der Freisetzungs- und Wirkungsort der DP IV-Inhibitoren durch die Art der Reste A-B gesteuert werden:

Im Blut von Säugern liegen neben der Dipeptidyl Peptidase IV verschiedene andere Aminopeptidasen wie z. B. Pyroglutamyl Aminopeptidase und Prolyl Aminopeptidase vor. Durch geeignete Auswahl der Reste A-B kann erfindungsgemäß festgelegt werden, durch welche Aminopeptidase der DP IV-Inhibitor freigesetzt werden soll und somit bestimmt werden, wo die Wirkung des Inhibitors eintreten soll. Die erfindungsgemäßen Prodrug-Verbindungen oder entsprechende pharmazeutische Zusammensetzungen können also auch zur zell-, gewebs- oder organspezifischen Inhibierung von DP IV eingesetzt werden. Die Gruppen A-B können auch so ausgewählt werden, daß nur solche Enzyme angesprochen werden, die nur vaskulär präsent sind und die die Inhibitoren mit ausreichend hoher Geschwindigkeit freisetzen.

Zusammenfassend kann festgestellt werden, daß durch die erfindungsgemäßen Prodrug-Verbindungen von DP IV-Inhibitoren in völlig übberaschender Weise:
1. Eine erhöhte Wirkung der Inhibitoren erreicht werden kann;
2. Die Freisetzung der Inhibitoren je nach Bedarf der Patienten erfolgen kann;
3. Die Freisetzung der Inhibitoren aus den Prodrug-Verbindungen zeitlich gesteuert erfolgen kann;
4. Die Freisetzung der Inhibitoren aus den Prodrug-Verbindungen bezüglich des Freisetzungsorts gesteuert werden kann; und
5. Ein Depot von DP IV-Inhibitoren bereitgestellt werden kann.

Erfindungsgemäß werden außerdem pharmazeutische Zusammensetzungen insbesondere zur oralen Verabreichung bereitgestellt, die dadurch gekennzeichnet sind, daß sie mindestens eine erfindungsgemäße Prodrug-Verbindung gegebenenfalls in Kombination mit üblichen Trägern oder Hilfsstoffen enthält. Die erfindungsgemäßen Prodrug-Verbindungen oder sie enthaltende pharmazeutischen Zusammensetzungen können zur Behandlung oder Prophylaxe von Erkrankungen von Säugern eingesetzt werden, die durch Modulation der DP IV-Aktivität eines Säugers behandelt werden können, wie z.B. von Stoffwechselerkrankungen von Menschen.

Insbesondere können sie zur Behandlung von beeinträchtigter Glukosetoleranz, Glukosurie, Hyperlipidämie, metabolischen Azidosen, Diabetes mellitus, diabetischer Neuropathie und Nephropathie sowie von durch Diabetes mellitus verursachten Folgeerkrankungen von Säugern eingesetzt werden.

### Beispiele

Die mit "⁺" gekennzeichneten Beispiele sind Referenzbeispiele, die nicht unter die Ansprüche fallen.

### 1. Synthese von erfindungsgemäßen Prodrugverbindungen

### 1.1. Synthese von H-Pro-Ile-Thia / HCl⁺

6,5 mM Boc-Pro-Ile-OH (ein Equivalent = 1 eq.) werden mit N-Hydroxybenzotriazol (1 eq.) und Thiazolidin (1 eq.) in 30 ml Dichlormethan (DCM) suspendiert. Bei -10 °C wird unter Rühren die equivalente Menge einer 1 M Dicyclohexylcarbodiimid-Lösung zugetropft. Man läßt bei -10 °C und über Nacht bei Raumtemperatur (RT) rühren. Zur Aufarbeitung wird gründlich vom ausgefallenen Dicyclohexylharnstoff abfiltriert, DCM im vakuum abgezogen und der erhaltene Rückstand in Essigester aufgenommen. Die Essigesterlösung wird mindestens dreimal mit gesättigter Bicarbonat-Lösung, einmal mit gesättigter NaCl-Lösung, dreimal mit verdünnter KHSO₄-Lösung und nochmals mit NaCl-Lösung gewaschen. Die Essigesterphase wird über Na₂SO₄ getrocknet, einrotiert und das verbleibende Rohprodukt mit Essigester/Pentan umkristallisiert. Boc-Pro-Ile-Thia kristallisiert nach 1-2 Tagen bei 4°C (Ausbeute 80%). Boc-Pro-Ile-Thia wird mit 1,1 N HCl/Eisessig-Lösung (3 ml pro mmol Peptid) versetzt. Man läßt zwei Stunden bei RT rühren, versetzt mit absolutem Ether und dampft überschüssige Abspaltungslösung am Rotationsverdampfer ab. Das Hydrochlorid kristallisiert quantitativ unter absolutem Ether über Nacht bei 4°C. Man saugt die Kristalle schnell unter mehrfachem Waschen mit absolutem Ether ab und bewahrt das Produkt im Ekksikator über KOH oder Phosphorpentoxid auf.

### 1.2. Synthese von H-Gly-Pro-Ile-Thia / HCl

Boc-Gly-OH (1 eq.) wird in 20 ml Tetrahydrofuran (THF) gelöst, auf -10 °C gekühlt und unter Rühren nacheinander mit N-Methylmorpholin (1 eq.) und Chlorameisensäureisobutylester (1 eq.) versetzt. Man läßt etwa 20 min aktivieren. Parallel dazu wird Pro-Ile-Thia x HCl (1 eq.) in 10 ml THF suspendiert auf -10 °C temperiert und mit N-Methylmorpholin (1 eq.) zur Neutralisation versetzt. Nach beendeter Aktivierungszeit werden beide Lösungen miteinander vermischt, nach ein bis zwei Stunden auf Raumtemperatur erwärmt und über Nacht gerührt. Danach wird das Reaktionsgemisch mit wenig Wasser versetzt und THF im Vakuum abdestilliert. Der verbleibende Rückstand wird in Essigester aufgenommen und mindestens dreimal mit gesättigter Natrium Bicarbonatlösung, einmal mit gesättigter NaCl-Lösung, dreimal mit verdünnter KHSO₄ und nochmals mit NaCl-Lösung gewaschen. Man trocknet die Essigesterphase über Na₂SO₄, rotiert ein und kristallisiert das Product Boc-Gly-Pro-Ile-Thiazolidid mit Essigester/Pentan um (Ausbeute 85 %). Die Boc-Abspaltung wird analog der Synthese von H-Pro-Ile-Thia / HCl durchgeführt (Ausbeute > 95 %).

**Tabelle 2: Analysedaten von Prodrugs von Inhibitoren der Dipeptidyl Peptidase IV**

| Substanz | MG berechnet* [g/mol] | MG gefunden M+H⁺ | CE Reinheit Retentionszeit (Rt) | HPLC Reinheit Rt | Schmelzpunkt °C |
|---|---|---|---|---|---|
| pGlu-Ile-Thia*HCl⁺ | 349,84 | 314,8 | 4,2 min | 10,4min | 30-40 |
| Pro-Ile-Thia*HCl⁺ | 335,90 | 300,8 | 4,5 min | 10,05 min | 45-69 |
| Gly-Pro-Ile-Thia*HCl | 392,94 | 357,8 | 4,6 min | 8,8 min | 111-121 |
| Ile-Pro-Ile-Thia*HCl | 449,05 | 413,6 | 5,6 min | 10,0 min | 98-107 |
| Pro-Pro-Ile-Thia*HCl | 433,01 | 397,6 | 5,3 min | 11,35 min | 101-118 |

| | | | | | |
|---|---|---|---|---|---|
| Bedingungen für die Analytik: HPLC Säule: LiChrospher 250-4, 100 RP-18,5µm, Temperatur 25°C Eluent: 30% ACN, 0,1% TFA, isokratisch, Flußgeschwindigkeit 0,5ml/min Detektionswellenlänge: 210nm CE Kapillare: 30cmx50µm fused silica, Temperatur 25°C Detektionswellenlänge: 200nm Injektion: 5sec, 50mbar Trennung: 0,1 M Na-Phosphatpuffer, pH 2,5; Dauer 7min bei 12kV | | | | | |

### 2. Affinität und Transport verschiedener Peptide, DP IV-Inhibitoren und Prodrugs zum Peptidtransporter PepT1

Die Affinität verschiedener Peptide, DP IV-Inhibitoren und Prodrugs von Inhibitoren der DP IV zum Peptidtransporter PepT1 wurde mittels Verdrängung des radioaktiv markierten Substrates D-Phe-Ala analysiert [AMASHEH, S., WENZEL, U., WEBER, W.M., CLAUSS, W., DANIEL, H., Electrophysiological analysis of the function of the mammalian renal peptide transporter expressed in Xenopus laevis oocytes. J. Physiol. 504, 169-174 (1997)]. Dabei zeigt sich, daß beispielsweise das Tetrapeptidderivat Ile-Pro-Ile-Thia vergleichbar bzw. besser als ausgewählte Aminosäure Derivate an das Transporterprotein PepT1 gebunden und im Vergleich zu ausgewählten Aminosäure- bzw. Peptidanaloga ähnlich oder besser transportiert wird (Tabelle 3).

**Tabelle 3: Transporteigenschaften verschiedener Aminosäure- und Peptidderivate am humanen Peptidtransporter PepT1**

| **Aminosäure-bzw. Peptidderivat** | **Elektrophys. Transportanalyse (hPEPT1 in Oocyten expr.), Flux % zur Kontr. Gly-Gln (100 %)** | **Bindungskonstante mM zu PepT1, relativ zu D-Phe-Ala** |
|---|---|---|
| Lys-Phe | 95 | 0,08 |
| Lys-Phe-Pro | 10 | 0,19 |
| Asn-Pyr | 30 | 3,01 |
| Asn-Thia | 83 | 0,50 |
| His-Pyr | 7 | 5,34 |
| His-Thia | 12 | 0,57 |
| Ile-Pyr | 14 | 2,66 |
| Ile-Thia | 25 | 0,98 |
| Ile-Pro-Ile-Thia | 44 | 0,61 |

### Freisetzung des aktiven DP IV-Inhibitors Ile-Thia aus erfindungsgemäßen Prodrugs in humanem Vollblut

Gemäß einer Ausführungsform von erfindungsgemäßen Prodrugs von DP IV-Inhibitoren ist auch eine retardierte Freisetzung von DP IV-Inhibitoren im Zielkompartiment, z.B. im Blutkreislauf möglich.

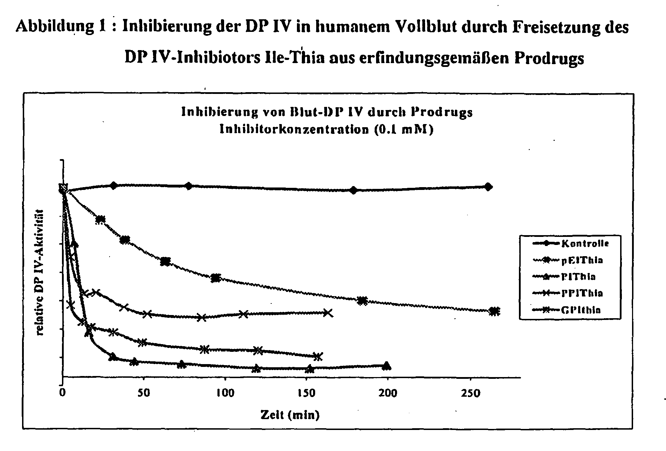

In Abbildung 1 ist die zeitabhängig differenziert verlaufende Inhibierung von humaner Blut-DP IV durch Freisetzung des Inhibitors Isoleucyl Thiazolidid aus erfindungsgemäßen Prodrug-Verbindungen exemplarisch dargestellt. Die Freisetzung des maskierten DP IV-Inhibitors im Blut kann im Falle der ausgewählten Beispiele (Pro-Pro-Ile-Thia = PPIThia, Gly-Pro-Ile-Thia = GPIThia) durch DP IV selbst bzw. durch Aminopeptidasen (pGlu-Ile-Thia⁺ = pEIThia, Pro-Ile-Thia⁺ = PIThia) erfolgen (Abbildung 1). Die Freisetzung des DP IV-Inhibitors Isoleucyl Thiazolidid aus den Prodrug-Verbindungen erfolgt im Blut bei gleicher eingesetzter Konzentration der Prodrugverbindung mit differenzierter Effizienz, wobei sich für Pro-Pro-Ile-Thia (PPI Thia) und pGlu-Ile-Thia⁺ (pEI Thia) eine im Vergleich zu Pro-Ile-Thia⁺ (PI Thia) und Gly-Pro-Ile-Thia (GPI thia) stärker retardierte Wirkstoff-Freisetzung zeigt.

### 3. Verstärkung der über DP IV-Inhibitoren vermittelten Glukosetoleranz durch den Einsatz von Prodrugs

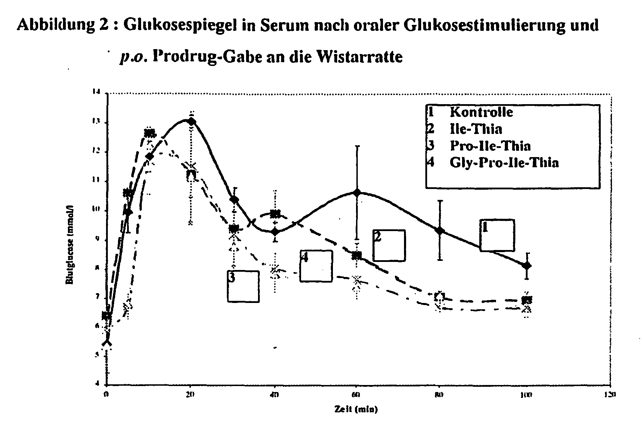

Durch Überführung des aktiven Wirkstoffes Isoleucyl Thiazolidid in erfindungsgemäße Prodrugs beobachtet man nach oraler Applikation bei der Wistar-Ratte ein deutlich verbessertes Wirkprofil (Abbildung 2). Die erwünschte Reduktion des Blutglukosespiegels durch DP IV-Inhibitoren im untersuchten Zeitintervall wird durch Einsatz der Prodrug-Verbindugen erfindungsgemäß gegenüber dem unmaskierten Wirkstoff Ile-Thia etwa um 30 % verstärkt (Tabelle 4):

**Tabelle 4: Abhängigkeit des Blutglukosespiegels innerhalb von 100 Minuten nach p.o. Glukosestimulierung und p.o. Verabreichung von Ile-Thia bzw. erfindungsgemäßen Prodrugs an Wistar-Ratten (Dosis: 2,5 µM Wirkstoff/300 g Tier)**

| **Wirkstoff / Prodrug** | **% Glukosespiegel** |
|---|---|
| Kontrolle | 100 |
| Ile-Thia | 74,4 |
| Gly-Pro-Ile-Thia | 57,1 |
| Pro-Ile-Thia⁺ | 56,1 |

## Patentansprüche

1. Prodrug-Verbindungen von Inhibitoren der Dipeptidyl Peptidase IV (DP IV), welche Prodrug-Verbindungen die allgemeine Formel A-B-C aufweisen, wobei
A eine Aminosäure
B Prolin, Hydroxyprolin, Thiazolidincarbonsäure, Dehydroprolin, Pipecolinsäure, Azetidincarbonsäure oder Aziridincarbonsäure, gebunden an A und C über Peptidbindungen, ist, und
C ein Aminoacylpyrrolidin-, Aminoacylthiazolidin- oder N-Dipeptidyl-O-acylhydroxylamin-Inhibitor von DP IV ist.

2. Prodrug-Verbindungen nach Anspruch 1, **dadurch gekennzeichnet, daß** B Prolin oder Hydroxyprolin ist.

3. Prodrug-Verbindungen nach Anspruch 1 oder 2, **dadurch gekennzeichnet, dass** A ausgewählt ist aus der Gruppe, bestehend aus Ile, Val, Phe, Pro, Ala und Gly.

4. Prodrug-Verbindungen nach einem der vorstehenden Ansprüche, **dadurch gekennzeichnet, daß** die Inhibitoren in Salzform vorliegen.

5. Prodrug-Verbindungen nach einem der vorstehenden Ansprüche, **dadurch gekennzeichnet, daß** A-B ein Dipeptid der Formel Ile-Pro oder Gly-Pro ist.

6. Pharmazeutische Zusammensetzung insbesondere zur oralen Verabreichung, **dadurch gekennzeichnet, daß** sie mindestens eine Prodrug-Verbindung nach einem der vorstehenden Ansprüche gegebenenfalls in Kombination mit üblichen Trägern oder Hilfsstoffen enthält.

7. Verwendung von Prodrug-Verbindungen oder pharmazeutischen Zusammensetzungen nach einem der Ansprüche 1 bis 6 für die Herstellung eines Medikaments zur Behandlung von beeinträchtigter Glukosetoleranz, Glukosurie, Hyperlipidämie, metabolischen Azidosen, Diabetes Mellitus, diabetischer Neuropathie und Nephropathie in Säugern.

8. Prodrug-Verbindung oder pharmazeutische Zusammensetzung nach einem der Ansprüche 1 bis 6 für die Verwendung bei der Behandlung von beeinträchtigter Glukosetoleranz, Glukosurie, Hyperlipidämie, metabolischen Azidosen, Diabetes Mellitus, diabetischer Neuropathie und Nephropathie in Säugern.

## Claims

1. Prodrug compounds of inhibitors of dipeptidyl peptidase IV (DP IV), which prodrug compounds have the general formula A-B-C, where
A is an amino acid
B is proline, hydroxyproline, thiazolidine-carboxylic acid, dehydroproline, pipecolic acid, azetidinecarboxylic acid or aziridinecarboxylic acid, attached to A and C via peptide bonds, and
C is an aminoacylpyrrolidine, aminoacylthiazolidine or N-dipeptidyl-O-acylhydroxylamine inhibitor of DP IV.

2. Prodrug compounds according to Claim 1, **characterized in that** B is proline or hydroxyproline.

3. Prodrug compounds according to Claim 1 or 2, **characterized in that** A is selected from the group consisting of Ile, Val, Phe, Pro, Ala and Gly.

4. Prodrug compounds according to any one of the preceding claims, **characterized in that** the inhibitors are present in salt form.

5. Prodrug compounds according to any one of the preceding claims, **characterized in that** A-B is a dipeptide of the formula Ile-Pro or Gly-Pro.

6. Pharmaceutical composition particularly for oral administration, **characterized in that** it contains at least one prodrug compound according to any one of the preceding claims optionally in combination with customary carriers or excipients.

7. Use of prodrug compounds or pharmaceutical compositions according to any one of Claim 1 to 6 in the manufacture of a medicament for treatment of impaired glucose tolerance, glucosuria, hyperlipidaemia, metabolic acidosis, diabetes mellitus, diabetic neuropathy and nephropathy in mammals.

8. Prodrug compound or pharmaceutical composition according to any one of Claims 1 to 6 for use in the treatment of impaired glucose tolerance, glucosuria, hyperlipidaemia, metabolic acidosis, diabetes mellitus, diabetic neuropathy and nephropathy in mammals.

## Revendications

1. Composés précurseurs de médicaments inhibiteurs de la dipeptidyle peptidase IV (DP IV), les composés de précurseurs médicaments présentant la formule générale A-B-C, où
A représente un acide aminé
B représente la proline, l'hydroxyproline, l'acide thiazolidinecarboxylique, la déshydroproline, l'acide pipécolique, l'acide azétidinecarboxylique ou l'acide aziridinecarboxylique, lié(e) à A et C via des liaisons peptidiques, et
C représente un inhibiteur de type aminoacylpyrrolidine, aminoacylthiazolidine ou N-dipeptidyl-O-acylhydroxylamine de la DP IV.

2. Composés précurseurs de médicaments selon la revendication 1, **caractérisés en ce que** B représente la proline ou l'hydroxyproline.

3. Composés précurseurs de médicaments selon la revendication 1 ou 2, **caractérisés en ce que** A est choisi dans le groupe constitué par Ile, Val, Phe, Pro, Ala et Gly.

4. Composés précurseurs de médicaments selon l'une quelconque des revendications précédentes, **caractérisés en ce que** les inhibiteurs se trouvent sous forme de sel.

5. Composés précurseurs de médicaments selon l'une quelconque des revendications précédentes, **caractérisés en ce que** A-B est un dipeptide de formule Ile-Pro ou Gly-Pro.

6. Composition pharmaceutique, en particulier pour l'administration par voie orale, **caractérisée en ce qu'**elle contient au moins un composé précurseur de médicaments selon l'une quelconque des revendications précédentes, le cas échéant en combinaison avec des supports ou des adjuvants usuels.

7. Utilisation de composés précurseurs de médicaments ou de compositions pharmaceutiques selon l'une quelconque des revendications 1 à 6 pour la préparation d'un médicament destiné au traitement d'une tolérance compromise au glucose, de la glucosurie, de l'hyperlipidémie, des acidoses métaboliques, du diabète sucré, de la neuropathie diabétique et de la néphropathie chez les mammifères.

8. Composé précurseur de médicaments ou composition pharmaceutique selon l'une quelconque des revendications 1 à 6 destiné(e) à être utilisé(e) lors du traitement d'une tolérance compromise au glucose, de la glucosurie, de l'hyperlipidémie, des acidoses métaboliques, du diabète sucré, de la neuropathie diabétique et de la néphropathie chez les mammifères.
